(19) Europäisches Patentamt
European Patent Office
Office européen des brevets

(11) Publication number: 0 057 542 B1

(12) EUROPEAN PATENT SPECIFICATION

(45) Date of publication of patent specification: 02.01.85

(51) Int. Cl.⁴: G 01 N 33/52, G 01 N 33/72

(21) Application number: 82300339.7

(22) Date of filing: 22.01.82

# (54) Improved specimen slide for occult blood testing.

(30) Priority: 29.01.81 US 229430

(43) Date of publication of application: 11.08.82 Bulletin 82/32

(45) Publication of the grant of the patent: 02.01.85 Bulletin 85/01

(84) Designated Contracting States: AT BE CH DE FR GB IT LI LU NL SE

(56) References cited:
EP-A-0 017 661
DE-A-2 716 060
GB-A-2 031 583
US-A-3 996 006

(73) Proprietor: SMITHKLINE INSTRUMENTS INCORPORATED
485 Potrero Avenue
Sunnyvale California 94086 (US)

(72) Inventor: Detweiler, Michael Burnside
2966 Moorpark Avenue NO. 32
San Jose California 95128 (US)
Inventor: Lawrence, Paul Joseph
2082 Abbey Lane
Campbell California 95008 (US)
Inventor: Townsley, Charles William
781 Foxridge Place
San Jose California 95133 (US)

(74) Representative: Johnston, Walter Paul et al
Patent Department SMITH KLINE & FRENCH LABORATORIES LTD Mundells
Welwyn Garden City Hertfordshire AL7 1EY (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 057 542 B1

Courier Press, Leamington Spa, England.

## Description

This invention relates to a specimen test slide for testing occult blood and a process for its preparation.

Specimen test slides and procedures for detecting occult blood in faecal matter are well known. For example, US Patent No. 3,996,006 discloses slides having a specimen receiving sheet between a front panel and a rear panel with openings in the front and rear panels and pivotal covers or flaps to cover these openings. One such test slide is sold under the trademark of "Hemoccult".

The specimen receiving sheet is generally an absorbent paper impregnated with a guaiac reagent. The haemoglobin catalyzed oxidation of the guaiac extract coated paper is used clinically to detect occult blood in faeces. Briefly, the test procedure is as follows.

A sample of faecal matter is smeared onto the guaiac paper through an opening of the front panel. The panel is then covered and the flap of the rear panel is opened. A developing solution such as hydrogen peroxide is applied to the guaiac paper via the corresponding opening in the rear panel. If blood is present in the faecal matter, the guaiac reaction will colour the paper blue. The overall reaction is as follows:

$$\underset{\text{Colourless}}{\text{Guaiac}} \xrightarrow[\text{Haemoglobin}]{H_2O_2} \underset{\text{Blue Colour}}{\text{"Oxidized" Guaiac}}$$

One of the major disadvantages of the guaiac test is that premature blueing occurs in the guaiac impregnated paper. Occasionally the paper will turn a slight blue colour upon exposure to air. Trace amounts of nitrogen dioxide, ozone, and other oxidants in the air can penetrate the closed slide and react with the guaiac in the paper to cause a blue colouration. This premature blueing can introduce an element of confusion in the test and result in false positive tests.

Previous attempts have been made to stabilize the guaiac impregnated paper. Some commercial test slides place glazing paper over the openings in the front panel to protect the guaiac paper when the slide is exposed to the atmosphere. German Offenlegungsschrift 2,716,060 discloses a method of treating the paper with various antioxidants. The results of this test revealed that the paper treated at protective anti-oxidant concentrations was not useful in detecting pathological amounts of blood in faeces because the sensitivity of the test was negatively influenced. When the concentrations of anti-oxidants were reduced so that a sufficient sensitivity of the test was reached, the substances lost their stablizing action.

Offenlegungsschrift 2,716,060 reported that of all the compounds tested, the 1-arylsemicarbazides, specifically 1-phenylsemicarbazide, gave some degree of protection when impregnated on the guaiac paper. However, 1-phenylsemicarbazide also increases the bleaching of the blue colour produced in a positive test.

Unexpectedly, it was discovered that when 2,6-di-tert-butyl-p-cresol was coated on the cover or flap of the slides described above and not impregnated in the guaiac paper, the paper was stable against premature blueing caused by light and air and pathological amounts of blood in faeces were easily detectable.

According to the present invention there is provided a specimen test slide comprising a specimen-receiving sheet carrying guiac sandwiched between a front and rear panel in which the slide can be opened to the front to expose a guaiac-carrying part of the sheet and to the rear, to expose the rear of that part of the sheet exposed to the front, and having 2,6-di-tert-butyl-p-cresol on at least part of the inner surface of that part of the slide overlying to the front or to the rear of the part of the sheet which is exposed.

The advantage of the slides of this invention is that the guaiac impregnated paper is prevented from blueing prematurely without essentially influencing the sensitivity of the test.

Several major advantages result in coating the panels instead of impregnating the guaiac paper with the anti-oxidants. Due to the small concentration of anti-oxidant required, the material can be applied by dissolving and/or suspending it in the thin film of varnish that is normally employed to prevent ink smearing during manufacture. Further, once the anti-oxidant is added to the coating varnish there is no change in the manufacturing procedure. This approach is very cost effective. Most important, since no additional material is introduced into the guaiac paper, the performance of the slide remains unchanged.

The slide can be opened to the front either because it has a portion which can be pulled away to expose the sheet below or because it has one or more openings through which the sheet is accessible, each of which has a cover.

Preferably the front panel has one or more (particularly two) openings each of which has a cover which can be opened to expose the sheet. Preferably there is a single cover for all of the openings. This cover can be hinged at one edge to the rear panel so as to swing closed over the openings.

The slide can be opened to the rear by, for example, a tear-off tab in the rear panel positioned so as to expose the rear of that part of the sheet exposed through the front panel. The panel can have a single tab which when open, exposes the sheet to the rear of all of the openings or can have a tab to the rear of each opening.

Preferably the rear panel has a single tab which when open, exposes the sheet to the rear of all the openings.

Preferably the tear-off tab is not completely detachable but is hinged along one edge to the rear panel.

By an inner surface of the slide is meant a surface adjacent the sheet.

Preferably the specimen receiving sheet is a sheet of absorbent paper.

Where the front panel has a number of openings, the slide has either a number of individual sheets, one underlying each opening, or a single sheet, a portion of which underlies each opening. Preferably the sheets are impregnated by printing with guaiac.

Adjacent sheets can be separated by a barrier which prevents the diffusion of liquid between sheets.

Preferably the panels and the cover consist of a sheet of foldable material folded into a middle section and two lateral flaps such that the middle section forms the rear panel and one flap folds over the middle to form the front panel and the other flap folds over the upper panel to form a cover.

An example of a test slide constructed as described above is sold under the Trade Mark "Hemoccult".

The 2,6-di-tert-butyl-p-cresol can be carried by the inner surface of the front or rear panel of the slide. Where the front panel has one or more openings with a cover or covers preferably the inner surface of the cover carries the 2,6-di-tert-butyl-p-cresol.

Where the rear panel has a tab, the 2,6-di-tert-butyl-p-cresol can be carried by the inner surface of the tab.

Preferably the 2,6-di-tert-butyl-p-cresol is present in a layer of varnish.

The 2,6-di-tert-butyl-p-cresol may be dissolved or suspended in a suitable organic solvent for example acetone or alcohol and applied to the cover flaps. Advantageously, varnish, which is a solution of a resin or drying oil in a volatile solvent, is employed as the carrier for the 2,6-di-tert-butyl-p-cresol. The varnish solution applied to the slide by coating or printing and not applied to the guaiac paper.

Preferably solutions or suspensions containing up to 5% of 2,6-di-tert-butyl-p-cresol may be employed. Most advantageously, solutions of about 2% to 3% are employed.

An embodiment of test slide in accordance with the invention will now be described with reference to the drawings where:

Fig. 1 is a perspective view from above of a slide according to the invention;

Fig. 2 is a perspective view from below of a slide of Fig. 1;

Fig. 3 is a plan view of a blank for making the slide of Figs. 1 and 2.

With reference to the drawings, the slide comprises an absorbent paper sheet carrying guaiac 1, sandwiched between a front panel 2, and a rear panel 3. The front panel 2 has two adjacent openings 4 and 5 through which the sheet is exposed to the front when the cover 6 is open. The cover 6 has a layer 7 (shaded), of 2,6-di-tert-butyl-p-cresol in, for example, a coating of varnish, on the side overlaying the openings that is adjacent the sheet. The slide has a cover 6 which is joined to the rear panel 3 by a hinge 8 on one of its edges. At the free edge remote from the hinged edge the cover has a tongue 9 which fits into a co-operating crescent shaped slot 10 which runs through the front and rear panels (to lock the cover).

The rear panel 3 has a tear-open tab 11 which when open, exposes the sheet to the rear of the openings in the front panel.

The slide of Figs. 1 and 2 is made up from a blank of Fig. 3. The blank is made of foldable material for example heavy paper or cardboard, creased along the lines 12 and 13 to form a middle portion or rear panel 3, one lateral flap or front panel 2 and another lateral flap or cover 6. The crease along 13 forms a hinge 8 joining the cover to the rear panel. Cover 6 terminates in a free edge remote from the hinged edge and this free edge has a tongue 9.

The sheet 1 is fixed on to the rear panel 3 with adhesive and the front panel 2 is fixed above the sheet by adhesive strips 2a. An adhesive spot 2b on the front panel which can be broken when the slide is to be used, holds cover closed after manufacture.

The layer of 2,6-di-tert-butyl-p-cresol can be applied as described in the following Examples which illustrate this invention.

Examples

Example 1

| Ingredients | Amounts |
|---|---|
| 2,6-di-tert-butyl-p-cresol | 10 g |
| Varnish | 454 g |

2,6-Di-tert-butyl-p-cresol was dissolved in the varnish and printed on the inner surface of the cover of a test slide of Figs. 1 to 3.

The varnish comprised a phenolic resin, China wood oil, linseed oil, and alcohol.

Example 2

| Ingredients | Amounts |
|---|---|
| 2,6-di-tert-butyl-p-cresol | 10 g |
| Acetone | 454 g |

2,6-Di-tert-butyl-p-cresol was dissolved in the acetone and coated on the inner surface of the cover of a slide Figs. 1 to 3.

Comparative tests

A number of anti-oxidants were tested for their ability to prevent premature blueing of slides of Figs. 1 to 3 by coating the slide, specifically the inside of the cover. The test procedure was as follows:

Six anti-oxidants were dissolved in acetone, 22.5 $\mu$moles of each were then applied to the inside of the cover. After all the solvent evaporated the slides were closed. The slides were then exposed to nitrogen dioxide in air, 2 ppm. The blueing time of each slide was noted. Following are the results:

| Compound | Blueing time* |
|---|---|
| Control (acetone alone) | 5 minutes |
| Vanillin | " |
| 1-phenylsemicarbazide | " |
| 4-phenylsemicarbazide | " |
| 4-phenyl-3-thiosemicarbazide | " |
| 3,3-thiodipropionic acid | " |
| 2,6-di-tert-butyl-p-cresol | 240 minutes |

The results clearly demonstrate that under exaggerated conditions, when 2,6-di-tert-butyl-p-cresol is coated on the slide cover, the guaiac paper is protected against blueing. It will be noted that although 1-phenylsemicarbazide has been reported to give some degree of protection when applied directly to the guaiac paper, it has no effect when coated on the inside of the cover of the slide.

2,6-Di-tert-butyl-p-cresol was dissolved in varnish, applied to the inside of the cover of a slide and the closed slide exposed to air under normal conditions. Following are the results of this test:

| mg 2,6-di-tert-butyl-p-cresol applied | Blueing time* |
|---|---|
| 0 | 2 days |
| 0.6 | 20 days |
| 1.5 | 27 days |

To use the slide the patient opens the cover 6 and applies a thin smear from one stool to sheet 1 through openings 4 and 5. He then closes the cover locking tongue 9 in slots 10 and returns the slide to his physician. On receiving the slide, the physician pulls open tab 11 and applies a reagent for example peroxide solution through the opening thus made, to the guaiac imgregnated sheet 1 opposite each of the openings 5 and 6 and observes the test results, that is the degree to which the colour blue is developed.

---

*Blueing time is defined as the time when the first tinge of blue is noted.

**Claims**

1. A specimen test slide comprising a specimen-receiving sheet carrying the guiac sandwiched between a front and rear panel in which the slide can be opened to the front, to expose a guaiac-carrying part of the sheet and to the rear, to expose the rear of that part of the sheet exposed to the front, and having 2,6-di-tert-butyl-p-cresol on at least part of the inner surface of that part of the slide overlying to the front or to the rear, the part of the sheet which is exposed.

2. A slide as claimed in claim 1, where the front panel has one or more openings each of which has a cover which can be opened to expose the sheet through the openings.

3. A slide as claimed in claim 2, having a single cover for all of the openings.

4. A slide as claimed in claim 2 or 3, in which the cover or covers carry the 2,6-di-tert-butyl-p-cresol.

5. A slide as claimed in any one of claims 1 to 4, where the rear panel has a tab which can be pulled away to expose the sheet.

6. A slide as claimed in claim 5, in which the tab carries the 2,6-di-tert-butyl-p-cresol.

7. A slide as claimed in any one of claims 1 to 6 where the 2,6-di-tert-butyl-p-cresol is in a layer of varnish.

8. A process for preparing a slide as claimed in any one of claims 1 to 7 which comprises applying a solution or suspension of 2,6-d-tert-butyl-p-cresol to a part of the inner surface of that part of the slide overlying to the front or to the rear, that part of the sheet which us exposed.

9. A process as claimed in claim 8 where the solution or suspension is applied to the cover or covers.

10. A process as claimed in claim 8 or claim 9 where the solution or suspension is in acetone or varnish.

11. A process as claimed in claim 10 where the solution or suspension is applied by printing.

**Patentansprüche**

1. Ein Proben-Teststreifen, der ein eine Probe aufnehmendes Blatt umfaßt, welches Guajakharz sandwichartig zwischen einer vorderen und einer rückwärtigen Platte enthält, wobei der Streifen auf der Vorderseite, um einen das Guajekharz enthaltenden Teil des Blattes freizulegen, und auf der Rückseite geöffnet werden kann, um die Rückseite des Teils des Blattes, der an der Vorderseite freigelegt ist, freizulegen, und der 2,6-Di-tert.-butyl-p-cresol auf mindestens einem Teil der inneren Oberfläche des Teils des Streifens aufweist, der über der Vorder- oder der Rückseite über dem Teil des Blattes liegt, der freigelegt ist.

2. Streifen nach Anspruch 1, wobei die vordere Platte eine oder mehrere Öffnungen aufweist, von denen jede eine Abdeckung hat, die geöffnet werden kann, um das Blatt durch die Öffnungen freizulegen.

3. Streifen nach Anspruch 2 mit einer einzigen Abdeckung für alle Öffnungen.

4. Streifen nach Anspruch 2 oder 3, wobei die Abdeckung oder Abdeckungen das 2,6-Di-tert.-butyl-p-cresol tragen.

5. Streifen nach einem der Ansprüche 1 bis 4, wobei die ruckwärtige Platte einen Lappen aufweist, der zur Freilegung des Blattes abgezogen werden kann.

6. Streifen nach Anspruch 5, bei dem der Lappen das 2,6-Di-tert.-butyl-p-cresol trägt.

7. Streifen nach einem der Ansprüche 1 bis 6, wobei sich das 2,6-Di-tert.-butyl-p-cresol in einer Lackschicht befindet.

8. Ein Verfahren zur Herstellung eines Streifens nach einem der Ansprüche 1 bis 7, welches das Aufbringen einer Lösung oder Suspension von 2,6-d-tert.-Butyl-p-cresol auf einen Teil der inneren Oberfläche des Teils des Streifens umfaßt, der von vorne oder von rückwärts über dem Teil des Blattes liegt, der freigelegt wird.

9. Verfahren nach Anspruch 8, wobei die Lösung oder Suspension auf die Abdeckung oder Abdeckungen aufgebracht wird.

10. Verfahren nach Anspruch 8 oder Anspruch 9, wobei die Lösung oder Suspension in Aceton oder Lack vorliegt.

11. Verfahren nach Anspruch 10, wobei die Lösung oder Suspension durch Drucken aufgebracht wird.

**Revendications**

1. Un porte-objet d'éprouvettes comprenant une feuille qui reçoit l'éprouvette et qui supporte du guaiac, intercalée entre un panneau avant et un panneau arrière, dans lequel le porte-objet peut être ouvert à l'avant, pour exposer une partie de la feuille supportant le guaiac et à l'arrière, pour exposer le dos de cette partie de la feuille exposée à l'avant, et ayant du 2,6-di-tert-butyl-p-crésol au moins sur une portion de la surface interne de cette partie du porte-objet dépassant vers l'avant ou vers l'arrière, partie de la feuille qui est exposée.

2. Un porte-objet comme indiqué dans la revendication 1, dans lequel le panneau avant a une ou plusieurs ouvertures dont chacune a une plaque protectrice qui peut être ouverte pour exposer la feuille, à travers les ouvertures.

3. Un porte-objet comme indiqué dans la revendication 2, ayant une seule plaque protectrice pour toutes les ouvertures.

4. Un porte-objet comme indiqué dans la revendication 2 ou 3 dans lequel la plague (les plaques) protectrice(s) supporte (nt) le 2,6-di-tert-butyl-p-crésol.

5. Un porte-objet comme indiqué dans n'importe laquelle des revendications de 1 à 4 dans lequel le panneau arrière a un onglet qui peut être tiré pour exposer la feuille.

6. Un porte-objet comme indiqué dans la revendication 5 dans lequel l'onglet supporte le 2,6-di-tert-butyl-p-crésol.

7. Un porte-objet comme indiqué dans n'importe laquelle des revendications de 1 à 6 dans lequel le 2,6-di-tert-butyl-p-crésol est une couche de vernis.

8. Un procédé pour la préparation d'un porte-objet comme indiqué dans n'importe laquelle des revendications de 1 à 7 et qui comprend l'application d'une solution ou suspension de 2,6-di-tert-butyl-p-crésol sur une partie de la face interne de cette partie du porte-objet qui recouvre le devant ou l'arrière, partie de la feuille qui est exposée.

9. Un procédé comme indiqué dans la revendication 8 par lequel la solution ou suspension est appliquée à la plaque protectrice ou les plaques protectrices.

10. Un procédé comme indiqué dans la revendication 8 ou 9, par lequel la solution ou suspension est de l'acétone ou du vernis.

11. Un procédé comme indiqué dans la revendication 10 par lequel la solution on suspension est appliquée par impression.

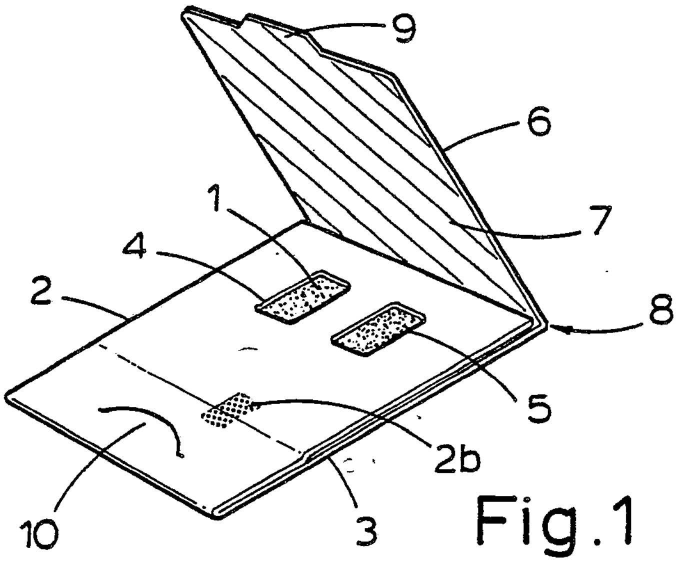


Fig.1

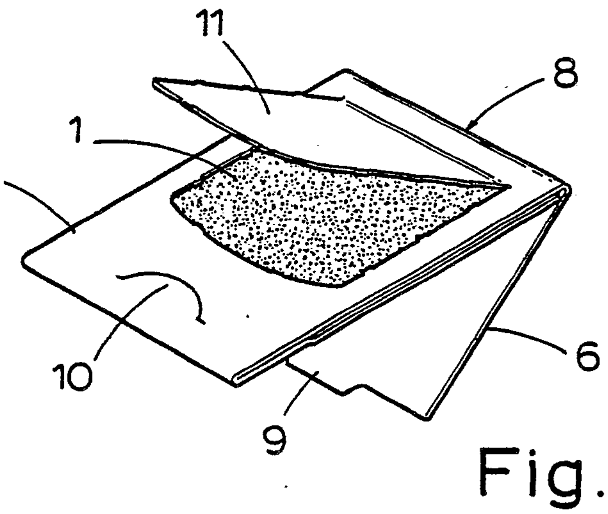


Fig.2

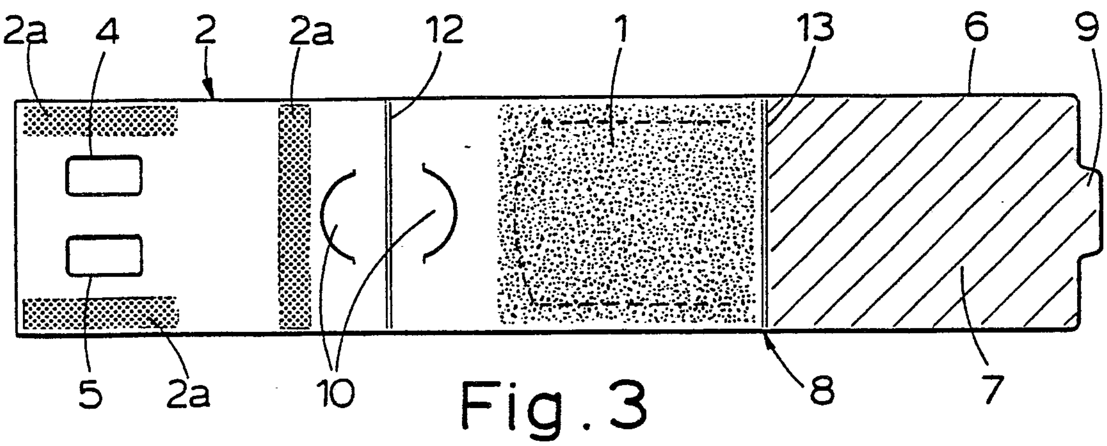


Fig.3